# EUROPEAN PATENT APPLICATION

(11) **EP 2 008 598 A1**
(43) Date of publication of application: **31.12.2008**
(21) Application number: 08153657.5
(22) Date of filing: 28.03.2008
(51) Int. Cl.: A61B 17/32, A61B 18/14, C22C 47/00

(54) **Composite fiber electrosurgical instrument**

(30) Priority: 29.06.2007 US 937651 P
(71) Applicant: Loeser, Edward A., Sandy, UT 84093-021 (US)
(72) Inventor: Loeser, Edward A., Sandy, UT 84093-021 (US)
(74) Representative: Hatzmann, Martin

(57) **Abstract**

Described is a surgical instrument for electrocauterization (e.g., an electric knife), the surgical instrument including: a body portion for holding and controlling the surgical instrument, and, physically attached thereto, a radio-opaque tip for cauterizing living body tissue, wherein at least the tip comprises a silicon material. In such an instrument, the tip is typically a tip electrode. Aspects of the invention may also comprise a tip (e.g., a tip electrode) made of a composite fiber and colored. Aspects of the invention may also comprise a tip made of at least one material selected from the group consisting of M60 pitch fiber, metal coated carbon fiber, metal impregnated into fiber, AS4 carbon fiber, polyacrylonitrile fiber, shaped vinyl carbon fiber, and combinations thereof.

## Description

### CROSS-REFERENCE TO RELATED APPLICATION

This application claims the benefit of U.S. Provisional Patent Appln. No. 60/937,651, filed on June 29, 2007, the contents of the entirety of which are incorporated by this reference.

### TECHNICAL FIELD

The present invention relates generally to surgical instruments and associated methods, and, more particularly, to electrosurgical instruments with advantageous material properties.

### BACKGROUND

Medical instrument coatings are applied in a variety of applications. One specialized type of medical instrument is an electrosurgical instrument such as an electric knife often referred to as an "RF scalpel."

Electrosurgical instruments are well known in the medical arts for incising soft patient tissue with concurrent transmission of electrical energy to the contacted tissue to cauterize small blood vessels and thereby minimize bleeding. Such instruments use a relatively high frequency electrical current passed through a typically stainless steel and conductive tool structure to disrupt blood vessels by vaporization and/or cauterization at the point of cutting contact. Such instruments typically comprise a body portion adapted for holding and manipulating the surgical instrument, wherein the body portion insulates an electrically conductive core.

Typically, electrosurgical instruments utilize an active electrode tip, sometimes also referred to as a "Bovie tip", to contact and cauterize the tissue. The tip is electrically conductive and cooperates with another conductor, such as a dispersive electrode (monopolar) or an adjacent electrode or tip (bipolar), to allow current flow at the site to be treated. These tips have a proximal end adapted to be mounted to an electric knife or other electrosurgical instrument by means of an insertion friction connection, threading, or other means, with the distal end defining an active metal electrode area of various to cut or cauterize tissue of interest. Typically, such electrode tips are specifically shaped according to intended use and vary in sharpness and thickness.

Electrosurgical instruments must effectively cut into and/or slide along the tissue and therefore, it is advantageous if not necessary to avoid the tendency of the distal end to stick on or to the tissue. Unfortunately, charred and necrosized tissue and cells can be generated by localized excessive thermal heating, wherein such tissue and cells tend to adhere to the surgical instrument such as the cutting edge of a surgical knife. The presence of such tissue and cells on the working surface of the instrument interferes with subsequent hemostatic cutting by disrupting the current field and correspondingly reducing the efficiency and efficacy of the instrument.

With the Bovie tip, a surgeon must be able to move the active electrode area along the tissue without carbonaceous remains from burnt tissue sticking thereto. Several proposals have been made to provide a non-stick surface to the active electrode area, however none is entirely satisfactory. For example, the tip may be coated with polytetrafluoroethylene (PTFE) or TEFLON® coating. TEFLON® material is not always easy to apply and may vaporize in use exposing the patient or surgeon to certain risks. Additionally, the TEFLON® coating is not a good conductor. Consequently, it has been the practice to expose the edges of the tip rather than provide the edges with the desirable nonstick coating. That, in turn, has limited the ability of the tip to conduct electricity uniformly across the surface. Furthermore, application of a TEFLON® coating requires additional processing time and cost.

Other examples are to plate the tip with platinum or coat the tip with conductive ceramic. The plating or coating process can be quite complex and costly. Indeed, platinum is itself quite costly. Relatedly, ceramics can be quite brittle, thus exposing the patient to risk of injury if pieces of ceramic chip or break off from the tip.

U.S. Patent 6,540,745 for "Coated Medical Devices", to Fairbourn and Loeser, discloses a silane coating applied to an electrode tip to facilitate non-stick and conductive properties. In addition, U.S. Patent 5,807,394 for "Surgical Instrument" to Sakai, further discloses carbon fibers incorporated into a carbon matrix creating a carbon/carbon composite material that may be coated onto a surgical tip to decrease buildup of carbonaceous adhesion during electrosurgery. Also, U.S. Patent 4,861,744 for "Thermoelectrically Controlled Heat Medical Catheter" to. Johnson et al., discloses using silicon with doped elements to produce thermoelectrically controlled heat. The contents of the entirety of these patents are incorporated herein by this reference.

Thus, a need exists for a medical device with a low cost, effective non-stick coating or material for tissue-contacting surfaces that retains desirable properties on an active electrode area.

### BRIEF SUMMARY OF THE INVENTION

Described herein is a relatively reliable, low cost alternative to traditional electrosurgical surfaces comprising various non-stick conductive materials without the drawbacks associated with platinum plating, ceramic coating, or TEFLON® coating. To this end, one aspect of the invention provides an electrosurgical instrument comprising at least a part formed from one or more non-stick conductive materials. In aspects of the invention, the conductive material may comprise a conductive thermoplastic, silicon, composite fibers, M60 pitch fiber, metal coated carbon fiber, AS4 carbon fiber, polyacrylonitrile fiber, shaped vinyl carbon fiber, polyimide, polyethylene sulfide (PES), polyetheretherketone (PEEK), and/or combinations thereof.

Aspects of the invention comprise a surgical instrument for electrocauterization (e.g., an electric knife), the surgical instrument comprising: a body portion for holding and/or controlling the surgical instrument, and, physically attached thereto, a tip for cauterizing living body tissue. In other aspects of the invention, at least a portion of the tip comprises a doped electro-conductive silicon material. In such an instrument, the tip may be a tip electrode.

Aspects of the invention may also comprise a surgical instrument (e.g., an electric knife), wherein the instrument comprises a body portion and a tip (e.g., a tip electrode) for contacting living body tissue, wherein at least a portion of the tip is made of a composite fiber. In additional aspects of the invention, the tip comprises a coloring agent. In other aspects of the invention, the tip may also comprise radio-opaque material.

Additional aspects of the invention may also comprise a surgical instrument (*e.g.,* an electric knife) comprising a body portion and a tip (e.g., tip electrode) for contacting living body tissue, wherein at least a portion of the tip comprises of at least one material selected from the group consisting of a composite fiber together with a coloring agent, a doped electro-conductive silicon material, a conductive thermoplastic, silicon, composite fibers, M60 pitch fiber, metal coated carbon fiber, metal impregnated into fiber, AS4 carbon fiber, polyacrylonitrile fiber, shaped vinyl carbon fiber, polyimide, polyethylene sulfide (PES), polyetheretherketone (PEEK), a coloring agent, radiopaque material, and combinations thereof.

In aspects of the invention, a portion of the tip may comprise silanes and/or a silane coating. In additional aspects the silane may be incorporated in the fiber matrix of the tip. In aspects of the invention, at least a portion of the tip may comprise antibiotics and/or anticlotting factors. In other aspects of the invention, at least a portion of the tip may comprise at least one coloring agent and/or radio-opaque material. In further aspects ofthe invention, the tip may further comprise a central wire within or on the surface of the tip for structural support and/or radio-opaqueness.

Aspects of the invention may also comprise a composite fiber tip for use with a surgical instrument for electrocauterization. In other aspects of the invention, the surgical instrument comprises a body portion for holding and/or controlling the surgical instrument, and, physically attached thereto, a composite fiber tip. In additional aspects of the invention, the composite fiber tip is used for cauterizing living body tissue. In further aspects of the invention, the composite fiber tip comprises a material that is electrically conductive, and thermally conductive. In other aspects of the invention, the tip thermo-conductivity is sufficiently high to enable the tip to reach temperatures sufficient to cauterize flesh. In additional aspects of the invention, the composite fiber tip may comprise at least one coloring agent. In other aspects of the invention, the composite fiber tip may comprise a radio-opaque material.

A surgical instrument, as described herein, may be incorporated into a kit comprising a body portion of a surgical instrument. In aspects of the invention, the kit comprises multiple color-coded tips for physical attachment with the body portion. In additional aspects of the invention, the multiple color-coded tips are of different sizes, shapes, and/or uses and may be received in a container. In other aspects of the invention, the container may comprise color-coded receptacles for receiving various color-coded tips.

Aspects of the invention may comprise a method of making a surgical instrument (e.g., an electric knife) for use in electrocauterization. In aspects of the invention, the method comprises making a body for holding and/or controlling the surgical instrument and attaching thereto a tip (e.g., tip electrode) for cauterizing living body tissue. In additional aspects of the invention, at least a portion of the tip comprises a doped electro-conductive silicon material.

Aspects of the invention may comprise a method of making a tip (e.g., tip electrode). In aspects of the invention, the method comprises incorporating at least one material selected from the group consisting of a composite fiber together with a coloring agent, a doped electro-conductive silicon material, a conductive thermoplastic, silicon, composite fibers, M60 pitch fiber, metal coated carbon fiber, metal impregnated into fiber, AS4 carbon fiber, polyacrylonitrile fiber, shaped vinyl carbon fiber, polyimide, polyethylene sulfide (PES), polyetheretherketone (PEEK), a coloring agent, radiopaque material, and combinations of any thereof.

Aspects of the invention also comprise a method of making a tip (e.g., tip electrode), the method comprising incorporating composite fiber into the composition of at least a portion of the tip. In aspects of the invention, the composite fiber tip comprises a material that is electrically conductive and thermally conductive. In aspects of the invention, the composite fiber tip may be colored with at least one coloring agent.

Aspects of the invention may comprise a method of electrocauterization, the method comprising cauterizing living body tissue with a surgical instrument according to the invention and, attaching thereto, a tip (e.g., a tip electrode) for cauterizing living body tissue. In other aspects of the invention, at least a portion of the tip comprises a doped electro-conductive silicon material.

Aspects of the invention may comprise a method of electrocauterization, the method comprising cauterizing living body tissue with a surgical instrument according to the invention and physically associated thereto, a tip (e.g., a tip electrode) for cauterizing living body tissue. In other aspects of the invention, at least a portion of the tip comprises composite fiber. In additional aspects of the invention, the composite fiber tip is electrically conductive and thermally conductive. In other aspects of the invention, the composite fiber tip may be colored with at least one coloring agent.

Aspects of the invention may comprise a method of electrocauterization, the method comprising cauterizing living body tissue with a surgical instrument according to the invention and, physically associated thereto, a tip (e.g., a tip electrode) for cauterizing living body tissue. In other aspects of the invention, at least a portion of the tip comprises at least one material selected from the group consisting ofM60 pitch fiber, metal coated carbon fiber, AS4 carbon fiber, polyacrylonitrile fiber, shaped vinyl carbon fiber, and combinations thereof

Aspects of the invention may comprise a method of making a kit, the method comprising securing at least one color-coded tip and at least one surgical instrument according to the invention within a container. In additional aspects of the invention, the container comprises color-coded receptacles for receiving color-coded tips. In other aspects of the invention, the surgical instrument comprises a body portion capable of attaching to at least one color-coded tip. In additional aspects of the invention, the color-coded tips may comprise different sizes, shapes, and/or uses

Aspects of the invention may also comprise a method of using a kit, the method comprising selecting at least one color-coded tip from a container for use with at least one surgical instrument according to the invention. In other aspects of the invention, the color-coded tips may be uprightly positioned in color-coded receptacles. In additional aspects of the invention, the surgical instrument may comprise a body portion capable of attaching to at least one color-coded tip.

Each aspect described herein may provide a non-stick conductive surface. Also, the aspects described herein may enable an electrothermally conductive surface. Furthermore, the aspects described herein may be less expensive to produce than platinum plating, ceramic coating, or TEFLON® coating.

### BRIEF DESCRIPTION OF THE SEVERAL VIEWS OF THE DRAWING

The accompanying drawings (not to scale), which are incorporated in and constitute a part of this specification, illustrate embodiments of the invention and, together with the general description of the given above, and the detailed description of the embodiments given below, serve to explain the principles of the present invention.

FIG. 1 is a view showing an example of an electrosurgical instrument used to cauterize living tissue.

FIG. 2 is a view showing an example of a monopolar tip (e.g., a tip electrode).

FIG. 3 is a view showing an example of a bipolar tip (e.g., a tip electrode).

FIG. 4 is a view showing an example of a kit comprising an electrosurgical instrument and a plurality of interchangeable tips (e.g., tip electrodes) of varying size, color, shape, and uses.

### DETAILED DESCRIPTION OF THE INVENTION

The invention will now be described below by way of non-limiting examples thereof with reference to the accompanying drawings.

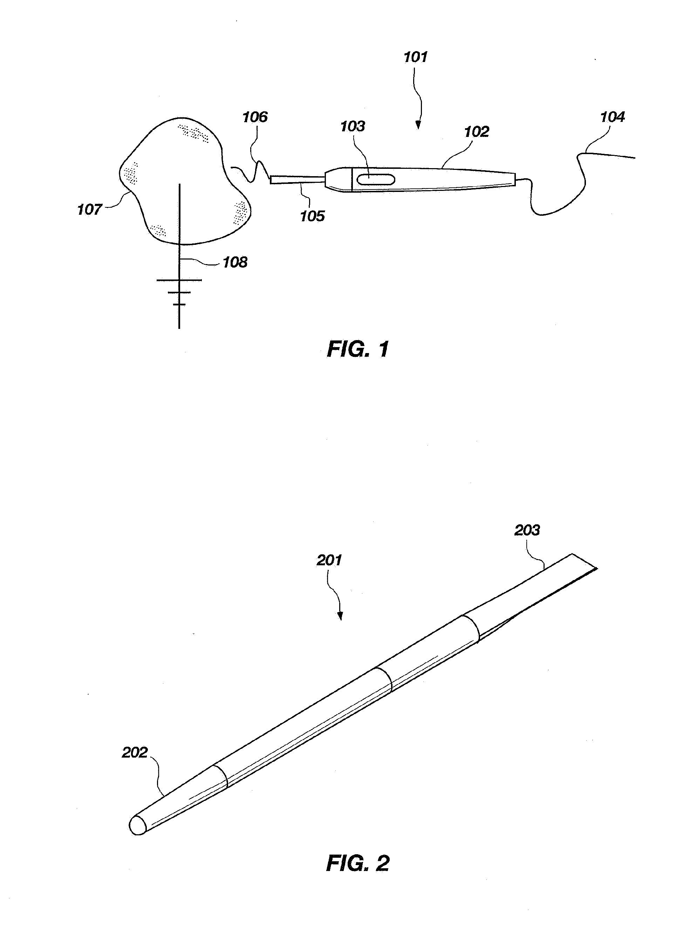

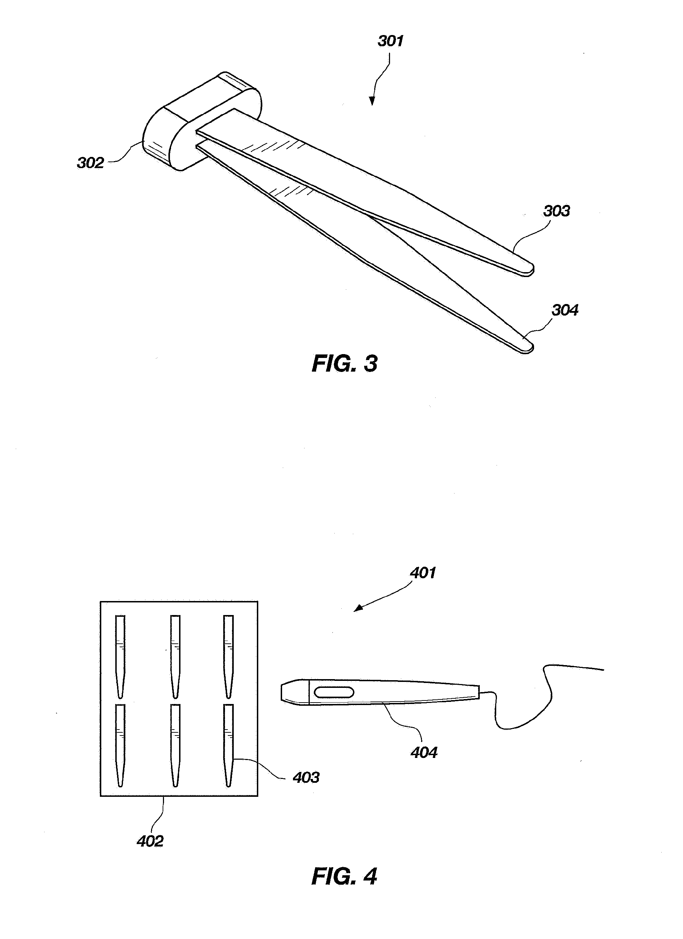

With reference to FIG.**1****,** there is shown a surgical instrument (*e*.*g*., an electric knife), generally designated at **101** and comprising an electric knife body portion **102** adapted for holding and manipulating the surgical instrument, wherein the body portion **102** insulates an electrically conductive core (not shown), a power control interface **103** for adjusting the intensity of electrical current flowing through the electrically conductive core, a power cord **104** intended to connect to an electric operating apparatus (not shown) and supply electric current to the electrically conductive core, and a tip (e.g., a tip electrode) **105** which at the distal end of the surgical instrument, wherein the tip can be monopolar (shown) or bipolar (not shown, see FIG. **3**) and receives current from the conductive core. In aspects of the invention, the tip **105** is physically associated with the knife body portion **102** by means of a friction connection insertion, threading, or other means. In aspects ofthe invention, the tip **105** may be shaped according to its desired function. In aspects of the invention, the tip **105** may comprise a sharp knife edge, a blunt tip, a blunt edge, paddle, hook, loop, or a ball shaped portion. In aspects of the invention, the tip may comprise curves at various angles to facilitate contact with desired tissue. In aspects of the invention, the thickness and mass of the tip **105** may also vary according to the intended use thereof.

In certain aspects of the invention, at least a portion of the tip **105** comprises a doped electro-conductive silicon material. In aspects of the invention, the electro-conductive silicon material may be deposited on the tip **105.** In aspects of the invention, the electro-conductive silicon material may be deposited on the tip **105** by means of sputtering and/or evaporating and depositing. In aspects of the invention, the tip **105** may comprise a radio-opaque material. This can be attained by adding a radiopaque substance such as barium sulfate, bismuth oxide, or tungsten. In aspects of the invention, the radio-opaque material may be a metal. In aspects of the invention, the tip **105** may further comprise at least one coloring agent. In additional aspects of the invention, the coloring agent may comprise metal particles and/or at least one silicon additive readily available in commercial settings.

In aspects of the invention, at least a portion of the tip 105 may be comprise at least one of a group of materials comprising a composite fiber together with a coloring agent, a doped electro-conductive silicon material, a conductive thermoplastic, silicon, composite fibers, M60 pitch fiber, metal coated carbon fiber, metal impregnated into fiber, AS4 carbon fiber, polyacrylonitrile fiber, shaped vinyl carbon fiber, polyimide, polyethylene sulfide (PES), polyetheretherketone (PEEK), a coloring agent, radiopaque material, and combinations thereof. In aspects of the invention, these materials have inherent non-stick properties and the productions costs associated with application of an additional TEFLON® coating are avoided. In aspects of the invention, the metal coating of a metal coated carbon fiber may comprise at least one material selected from the group consisting of copper, nickel, zinc, and stainless steel.

A block-shaped composite material disclosed in Japanese Patent Gazette No. 2000-223004 is formed by mixing fine carbon fibers with metal powders and sintering the mixture. *See, also,* US Patent Application 20060021877, US Patent Application 20070141315 (Metal-based carbon fiber composite material and method for producing the same), and JP 2003-40308, the contents of the entirety of each of which are incorporated by this reference. In certain embodiments, metal was added to carbon fibers by adding it to the resin or sprinkling it within "prepreg" fibers (fibers that had the resin already applied) in preparation for molding and/or heating the material to give it a permanent shape. Copper, nickel, etc. was added by just dusting it onto the carbon fiber, and then making a tip in a mold shaped as desired.

In additional aspects of the invention, metal coated fiber is preferably radio-opaque so as to enable identification of the tip by, for example, x-ray. In aspects of the invention, the metal coated fiber may provide increased conductivity in comparison to traditional carbon fiber. Also, at least a portion of the tip **105** may comprise nano sized cross linked fibers. In addition aspects of the invention, the nano sized cross linked fibers may connect the material within the tip **105.** In aspects of the invention, the nano sized cross linked fibers may increase the conductivity of the tip **105.** In other aspects of the invention, at least a portion of the tip **105** may comprise silanes and/or a silane coating. In additional aspects of the invention, silane may also be incorporated in the fiber matrix of the tip. In other aspects of the invention, silane may inhibit the buildup of carbonaceous material on the tip **105.** In additional aspects of the invention, at least a portion of the tip **105** may also comprise antibiotics and or anticlotting factors. In other aspects of the invention, anticlotting factors may comprise of heparin. In additional aspects of the invention, antibiotics and anticlotting factors may facilitate wound healing and fight infection. In other aspects of the invention, antibiotics and anticlotting factors may be able to withstand the heat generated during the electrosurgical process. In additional aspects of the invention, the tip **105** may also comprise at least one coloring agent and/or radio-opaque material. In other aspects of the invention, coloring agents may comprise dyes, color coatings, paints, and/or colored metal particles that are readily available in commercial settings. In additional aspect of the invention, metal particles may also be radio-opaque. In other aspects of the invention, the tip **105** may comprise a central wire (not shown) within or on the surface of the tip for structural support. In other aspects of the invention, the central wire may be radio-opaque. In additional aspects of the invention, the central wire may be used to identify the tip **105** under radiographic and/or ultrasound guidance (e.g., robotically controlled neurosurgical procedures).

In aspects of the invention, at least the tip **105** may comprise composite fiber that is electrically conductive and thermally conductive. In additional aspects of the invention, the composite fiber may be colored with at least one coloring agent. In other aspects of the invention, coloring agents may comprise dyes, color coatings, paints, and/or colored metal particles that are readily available in commercial settings. In additional aspects of the invention, the coloring agent may be incorporated into the composite matrix of the composite fiber tip. In other aspects of the invention, the tip **105** may further comprise a radio-opaque material. In additional aspects of the invention, the radio-opaque material may be a metal.

The tip **105** of the surgical instrument **101** may be used in electrocauterization, wherein an electric current arc **106** is used to cauterize living tissue **107.** At least in the monopolar embodiment, the living tissue may be electrically grounded **108** and a second electrode (not shown) may be in direct contact with the patient's tissue.

With reference to FIG. 2, there is shown a monopolar tip (e.g., a tip electrode) **201** comprising a proximal end **202** for attachment to a surgical instrument (e.g., an electric knife) instrument by means of insertion, threading, or other means, and a distal end **203** for contacting and cauterizing living tissue. The monopolar tip **201** may be shaped according to its desired function. In aspects of the invention, the monopolar tip **201** may comprise a sharp knife edge, a blunt tip, a blunt edge, paddle, hook, loop, a ball shaped portion, or another shape. In aspects of the invention, the tip may be curved at various angles to facilitate contact with desired tissue. The thickness and/or mass of the monopolar tip **201** may also vary according to the intended use thereof. In aspects of the invention, the monopolar tip **201**may comprise at least one aspect of the FIG. **1** tip **105.**

With reference to FIG. 3, there is shown a bipolar tip (e.g., a tip electrode) **301** comprising a proximal end **302** for attachment to a surgical instrument (e.g., an electric knife) instrument by means of insertion, threading, or other means, and two distal ends **303** and **304** for contacting and cauterizing living tissue. In other aspects of the invention, the bipolar tip **301** may be shaped according to its desired function. In aspects of the invention, the bipolar tip **301** may comprise a sharp knife edge, a blunt tip, a blunt edge, paddle, hook, loop, or a ball shaped portion, or a another shape. In aspects of the invention, the tip may be curved at various angles to facilitate contact with desired tissue. In aspects of the invention, the thickness and/or mass of the bipolar tip **301** may also vary according to the intended use thereof. In aspects of the invention, the two distal ends **303** and **304** may also be shaped according to indented use. In aspects of the invention, distal ends **303** and **304** may be shaped differently from the other. In aspects of the invention, the bipolar tip **301** may comprise at least one aspect of the FIG. **1** tip **105.**

With reference to FIG. **4****,** there is shown a kit **401** comprising a container **402** for securing at least one color-coded tip **403** for receipt within the body portion of a surgical instrument (e.g., an electric knife) **404.** In aspects of the invention, the color-coded tips **403** may vary in size, shape, and use. In aspects of the invention, the container **402** may comprise color-coded receptacles (not shown) specifically shaped for receiving at least one color-coded tip. In aspects of the invention, the container **402** may also comprise at least one receptacle for receiving at least one surgical instrument. In aspects of the invention, the entire kit **401** may be sterilized as a whole. In additional aspects of the invention, a color-coded tip **403** may be attached to a surgical instrument while the color-coded tip remains in its receptacle, thus avoiding any handling of the tip. This arrangement may facilitate operating room procedures and may avoid contamination through handling of the tip.

Aspects of the invention may comprise a method of making a surgical instrument **101** (e.g., an electric knife) for use in electrocauterization. In aspects of the invention, the method comprises making a body **102** for holding and/or controlling the surgical instrument **101** and attaching thereto a tip **105** *(e.g.,* tip electrode) for cauterizing living body tissue **107.** In aspects of the invention, the method may comprise adding at least one coloring agent as described herein. In other aspects of the invention, the method may comprise adding at least one radio-opaque material as described herein

Aspects of the invention may comprise a method of making a tip **105** *(e.g.,* tip electrode). In aspects of the invention, the method comprises incorporating at least one material selected from the group consisting of M60 pitch fiber, metal coated carbon fiber, AS4 carbon fiber, polyacrylonitrile fiber, shaped vinyl carbon fiber, and combinations thereof into the composition of at least a portion of the tip **105.** In aspects of the invention, the method may comprise adding at least one coloring agent as described herein. In other aspects of the invention, the method may comprise adding at least one radio-opaque material as described herein.

Aspects of the invention also comprise a method of making a tip **105** *(e.g.,* tip electrode), the method comprising incorporating composite fiber into the composition of at least a portion of the tip **105.** In aspects of the invention, the composite fiber tip comprises a material that is electrically conductive and thermally conductive. In aspects of the invention, the composite fiber tip may be colored with at least one coloring agent. In aspects of the invention, the coloring agent may comprise dyes, color coatings, paints, and/or colored metal particles that are readily available in commercial settings.

Aspects of the invention may comprise a method of electrocauterization, the method comprising cauterizing **106** living body **107** with a surgical instrument **101** according to the invention, and, attaching thereto, a tip **105** *(e.g.,* a tip electrode) for cauterizing living body tissue. In aspects of the invention, at least a portion of the tip **105** comprises a doped electro-conductive silicon material.

Addition aspects of the invention may comprise a method of electrocauterization, the method comprising cauterizing **106** living body tissue **107** with a surgical instrument **101** according to the invention and, physically associated thereto, a tip **105** (e.g., a tip electrode) for cauterizing living body tissue. In aspects of the invention, at least a portion of the tip **105** comprises composite fiber. In additional aspects of the invention, the composite fiber tip is electrically conductive and thermally conductive. In other aspects of the invention, the composite fiber tip may be colored with at least one coloring agent as described herein.

Aspects of the invention may comprise a method of electrocauterization, the method comprising cauterizing **106** living body tissue **107** with a surgical instrument **101** according to the invention and, physically associated thereto, a tip **105** (e.g., a tip electrode) for cauterizing living body tissue. In aspects of the invention, at least a portion of the tip **105** comprises at least one material selected from the group consisting of a composite fiber together with a coloring agent, a doped electro-conductive silicon material, a conductive thermoplastic, silicon, composite fibers, M60 pitch fiber, metal coated carbon fiber, metal impregnated into fiber, AS4 carbon fiber, polyacrylonitrile fiber, shaped vinyl carbon fiber, polyimide, polyethylene sulfide (PES), polyetheretherketone (PEEK), a coloring agent, radiopaque material, and combinations thereof.

Aspects of the invention may comprise a method of making a kit **401,** the method comprising securing at least one color-coded tip **403** and at least one surgical instrument **404** within a container **402.** In aspects of the invention, the container **403** comprises color-coded receptacles (not shown) for receiving color-coded tips. In aspects of the invention, the surgical instrument **402** comprises a body portion capable of attaching to at least one color-coded tip **403.** In aspects of the invention, the color-coded tips may be attached to the surgical instrument while the color-coded tip remains in its receptacle, thus avoiding any handling of the tip. This arrangement may facilitate operating room procedures and may avoid contamination through handling of the tip. In aspects ofthe invention, the color-coded tips may comprise different sizes, shapes, and/or uses. In aspects of the invention, the entire kit **401** may be sterilized as a whole.

Aspects of the invention may also comprise a method of using a kit **401,** the method comprising selecting at least one color-coded tip **403** from a container **402** for use with at least one surgical instrument **404.** In aspects of the invention, the color-coded tips **403** may be uprightly positioned in color-coded receptacles (not shown). In aspects of the invention, the surgical instrument may comprise a body portion capable of attaching to at least one color-coded tip. In additional aspects of the invention, a color-coded tip may be attached to the surgical instrument while the color-coded tip remains in its receptacle, thus avoiding any handling of the tip. This arrangement may facilitate operating room procedures and may avoid contamination through handling of the tip. In additional aspects of the invention, the color-coded tips may comprise different sizes, shapes, and/or uses. In aspects of the invention, the entire kit **401** may be sterilized as a whole.

Each aspect described herein may provide a non-stick conductive surface. Also, the aspects described herein may enable an electrothermally conductive surface. Furthermore, the aspects described herein may be less expensive to produce than platinum plating, ceramic coating, or TEFLON® coating.

While this invention has been described in certain aspects, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A surgical instrument for electrocauterization comprising:
a body portion for holding and manipulating the surgical instrument, and, physically associated therewith, and
a tip for cauterizing living body tissue and comprising at least one radio-opaque material,
wherein at least a portion of the tip is made of at least one material selected from the group consisting of a composite fiber together with a coloring agent, a doped electro-conductive silicon material, a conductive thermoplastic, silicon, composite fibers, M60 pitch fiber, metal coated carbon fiber, metal impregnated into fiber, AS4 carbon fiber, polyacrylonitrile fiber, shaped vinyl carbon fiber, polyimide, polyethylene sulfide (PES), polyetheretherketone (PEEK), a coloring agent, and/or combinations thereof.

2. The surgical instrument of claim 1, wherein the tip is a tip electrode.

3. The surgical instrument of claim 1, wherein at least a portion of the tip further comprises silane.

4. The surgical instrument of claim 1, wherein at least a portion of the tip further comprises an antibiotic anticlotting factor.

5. The surgical instrument of claim 1, wherein the tip further comprises a central support.

6. The surgical instrument of claim 5, wherein the central support is a wire.

7. The surgical instrument of claim 1, wherein at least a portion of the tip further comprises nano sized cross linked fibers.

8. The surgical instrument of claim 2, wherein the surgical instrument is an electric knife.

9. The surgical instrument of claim 1, wherein the tip comprises a metal impregnated into fiber.

10. A kit comprising:
a body portion of the surgical instrument of claim 1,
multiple tips for receipt within the body portion, wherein each tip is a separate color and is radio-opaque, and
a container comprising a receptacle structure for securing the body portion and tips.

11. A composite fiber tip for use with a surgical instrument for electrocauterization, wherein the surgical instrument comprises a body portion for holding and manipulating the surgical instrument, and
wherein the composite fiber tip is a radio opaque tip electrode physically associated with the body portion, and comprises a material that is electrically conductive, thermally conductive, and colored with at least one added coloring agent incorporated therein, and wherein at least a portion of the tip is made of at least one material selected from the group consisting of a composite fiber together with a coloring agent, a doped electro-conductive silicon material, a conductive thermoplastic, silicon, composite fibers, M60 pitch fiber, metal coated carbon fiber, metal impregnated into fiber, AS4 carbon fiber, polyacrylonitrile fiber, shaped vinyl carbon fiber, polyimide, polyethylene sulfide (PES), polyetheretherketone (PEEK), a coloring agent, and/or combinations thereof.

12. The composite fiber tip of claim 11, wherein the surgical instrument is an electric knife.

13. The composite fiber tip of claim 11, wherein the material comprises metal impregnated into fiber.

14. Use of the surgical instrument of claim 1 for electrocauterization.

15. A kit comprising:
the surgical instrument of claim 1, wherein the surgical instrument comprises a body portion capable of receiving at least one colored tip,
at least one colored, radio-opaque tip, and
a container,
wherein the container comprises a receptacle of the same color as the colored tip and is specifically formed for receiving at least one colored tip.

## Amended claims

### Amended claims in accordance with Rule 137(2) EPC.

**1.** A surgical instrument for electrocauterization comprising:
a body portion for holding and manipulating the surgical instrument, and, physically associated therewith, and
a tip for cauterizing living body tissue and comprising at least one radio-opaque material,
**characterized in that**
at least a portion of the tip is made of at least one electrically conducting material selected from the group consisting of a composite fiber together with a coloring agent, a doped electro-conductive silicon material, a conductive thermoplastic, silicon, composite fibers, M60 pitch fiber, metal coated carbon fiber, metal impregnated into fiber, A34 carbon fiber, polyacrylonitrile fiber, shaped vinyl carbon fiber, polyimide, polyethylene sulfide (PES), polyetheretherketone (PEEK), a coloring agent, and/or combinations thereof.

**2.** The surgical instrument of claim 1, wherein the tip is a tip electrode.

**3.** The surgical instrument of claim 1, wherein at least a portion of the tip further comprise silane.

**4.** The surgical instrument of claim 1, wherein at least a portion of the tip further comprises an antibiotic anticlotting factor.

**5.** The surgical instrument of claim 1, wherein the tip further comprises a central support.

**6.** The surgical instrument of claim 5, wherein the central support is a wire.

**7.** The surgical instrument of claim 1, wherein at least a portion of the tip further comprises a nano cross linked fibers.

**8.** The surgical instrument of claim 2, wherein the surgical instrument is an electric knife.

**9.** A kit comprising:
a body portion of the surgical instrument of claim 1,
multiple tips for receipt within the body portion, wherein each tip is a separate color and radio-opaque, and
a container comprising a receptacle structure for securing the body portion and tips.

**10.** A composite fiber tip for use with a surgical instrument according to claim 1.

**11.** The composite fiber tip according to claim 10, wherein the surgical instrument is an electric knife.

**12.** A kit according to claim 9, wherein the receptacle is of the same color as the colored tip and is specifically formed for receiving at least one colored tip.
